# EUROPEAN PATENT SPECIFICATION

(11) **EP 0 357 403 B1**
(45) Date of publication and mention of the grant of the patent: **14.12.1994**
(21) Application number: 89308779.1
(22) Date of filing: 30.08.1989
(51) Int. Cl.: C07C 49/248, C07C 49/235, C07C 49/255, C07C 49/213, C07C 49/217, C07C 49/203, C07D 333/22, C07D 307/46, C07C 255/56, A61K 31/12

(54) **Substituted beta-diketones**
Substituierte beta-Diketone
Bêta-dicétones substituées

(30) Priority: 01.09.1988 GB 8820729
(43) Date of publication of application: 07.03.1990
(73) Proprietor: ORION-YHTYMÄ OY, 02101 Espoo (FI)
(72) Inventor: Pohto, Pentti, SF-00530 Helsinki (FI); Aho, Paivi Annikki, SF-00100 Helsinki (FI); Backstrom, Reijo Johannes, SF-00750 Helsinki (FI); Honkanen, Erkki Juhani, SF-01400 Vantaa (FI); Linden, Inge-Britt Yvonne, SF-00210 Helsinki (FI); Nissinen, Erkki Aarne Olavi, SF-02170 Espoo (FI)
(74) Representative: Sexton, Jane Helen

(56) References cited:
- CH-A- 629 741
- DE-A- 2 155 495
- US-A- 3 998 872
- US-A- 4 153 719
- US-A- 4 456 770
- CHEMICAL ABSTRACTS, vol. 108, no. 17, April 25, 1988, Columbus, Ohio, USA; M. JIANG et al, "Comparison of homologous graduation for isosteric thienyl- and phenylpolyene series", page 678, abstract-no. 149 798x
- CHEMICAL ABSTRACTS, vol. 96, no. 25, June 21, 1982 Columbus, Ohio, USA; E. K. POHJALA et al, "Electron-impact mass spectra of 2-(2-pyridyl)methylene-1,3-dicarbonyl compounds. Unusual abundance of the [M+1]+ ion and the effect of stereochemistry", page 667, abstract-no. 217 042g
- CHEMICAL ABSTRACTS, vol. 96, no. 23, June 7, 1982, Columbus, Ohio, USA; E. SOLCANIOVA et al, "Substituent effects on carbon-13 and proton chemical shifts in 3-benzylidene-2,4-pentanediones", page 603, abstract-no. 198 944j
- CHEMICAL ABSTRACTS, vol. 83, no. 11, September 15, 1975, Columbus, Ohio, USA; M. YAMASHITA et al, "Reaction of tetracarbonylhydridoferrate with the Knoevenagel condensates of 2,4-pentanediones with aldehydes. New synthetic route to alkyl methyl ketones from aldehydes", page 502, abstract-no. 96 338d

## Description

The present invention relates to β-diketones and their physiologically acceptable salts, esters and ethers, and pharmaceutical compositions containing these compounds which are useful as cytoprotective agents and in particular as antiulcerogenic or gastroprotective agents.

British patent application no. 8730190 discloses a group of compounds including some β-diketones such as 3-(3,4-dihydroxy-5-nitrobenzylidene)-2,4-pentadione, which are useful as agents for the treatment or prophylaxis of ulcers, lesions or like condition in the gastrointestinal tract. It has now been found that other β-diketones are effective as cytoproptective agents, and in particular are useful in the treatment or prophylaxis of ulcers, lesions or like condition in the gastrointestinal tract.

CH-A-629741 describes arylenedioxy-bis-diacetones as anti-viral agents.

US-A-4153719 describes β-diketones substituted by an aryl-aliphatic group in which the aliphatic chain is interrupted by a cyclic group. These compounds are described as anti-viral agents.

US-A-4456770 describes (3',5'-dihydrocarbyl-4'-hydroxybenzyl)-1,3-diketones which are useful as antioxidants for oxidisable organic materials when such materials are exposed to oxidative degradative conditions.

US-A-3998872 describes α-substituted allylic carbonyl compounds as exemplified by 3-(1-methylallyl)-2,4-pentanedione. The compounds are described as being useful intermediates in preparing chemical compounds which possess desirable fragrances.

DE-A-2155495 describes a process for preparing benzylidene compounds from malonic acid esters and benzyl halides. The compounds produced are of the formula: 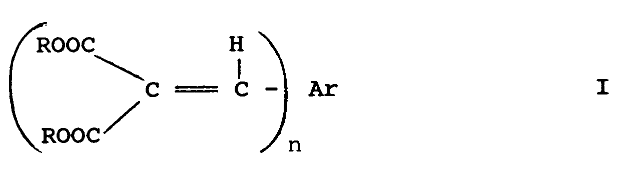
where R is a straight chain or branched alkyl residue of 1 to 20 carbon atoms or an aryl residue; Ar is an aromatic or heteroaromatic residue such as phenyl or naphthyl optionally substituted by one or more substituents and n is from 1 to 4. The compounds are said to find use as UV absorbers.

CA-108 149798x describes thienylpolyenic compounds in which the thienyl group is unsubstituted. No utility is described for these compounds.

CA-96 217042g describes the electron-impact mass spectra of 2-(2-pyridyl)methylene-1,3-dicarbonyl compounds. No utility is described for these compounds.

CA-96 198944j describes substituent effects on carbon-13 and proton chemical shifts in 3-benzylidene-2,4-pentanediones. No utility is described for these compounds.

CA-83 96338d describes the reaction of tetracarbonyl hydridoferrate with the Knoevenagel condensates of 2,4-pentanediones with aldehydes to give compounds of formula RCH:C(COMe)₂ where R is propyl, isopropyl, ethyl, benzyl or 2-furyl. No utility is described for these compounds.

The present invention provides a compound which is of the formula Ib: 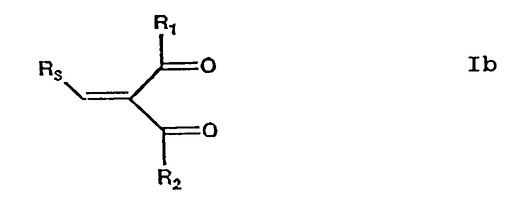
wherein R₁ and R₂ are independently C₁₋₆ alkyl which is unsubstituted or substituted by hydroxy, C₁₋₆ alkoxy, halogeno, nitro or amino; C₁₋₆ alkoxy; phenyl or naphthyl which is unsubstituted or substituted by C₁₋₆ alkyl, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
R₃ is C₁₋₆ alkyl which is substituted by hydroxy, C₁₋₆ alkoxy, halogeno, nitro or amino; phenyl or naphthyl which are substituted by trifluoromethyl or cyano; or one of the following heteroaryl groups: quinolyl, thienyl or furyl which are substituted by C₁₋₆ alkyl, hydroxy, C₁₋₆ alkoxy, halogeno, nitro or amino;
or a salt, ether or ester thereof, provided that when R₃ is substituted furyl it is not substituted by alkyl, halogeno or nitro and if R₃ is a phenyl which is substituted by cyano then the cyano is not in the para position.

The optional substituent of an alkyl group is hydroxy, alkoxy of 1 to 6 carbon atoms, halogeno, nitro or amino, and preferably the alkyl group does not carry more than three substituents.

In the compounds of the invention the carbon carbon double bond forms a conjugated system of double bonds with the keto groups.

Preferred compounds include those in which R₁ and R₂ are each C₁₋₄ alkyl and R₃ is thienyl, fury], or phenyl which is substituted by hydroxy, C₁₋₄ alkoxy, trifluoromethyl or cyano, the phenyl group having at least two substituents, or R₃ is carboxyphenyl.

The present invention also provides a compound of formula Ib 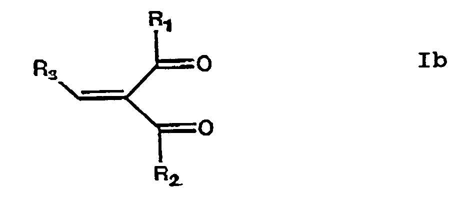
wherein R₁ and R₂ are independently C₁₋₆ alkyl which is unsubstituted or substituted by hydroxy, C₁₋₆ alkoxy, halogeno, nitro or amino; C₁₋₆ alkoxy; phenyl or naphthyl which is unsubstituted or substituted by C₁₋₆ alkyl, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
R₃ is C₁₋₆ alkyl which is unsubstituted or substituted by hydroxy, C₁₋₆ alkoxy, halogeno, nitro or amino; phenyl or naphthyl which are unsubstituted or substituted by C₁₋₆ alkyl, C₁₋₆ alkoxy, trifluoromethyl, cyano, amino or carboxy or a diketo group attached to the aryl group directly or via a saturated hydrocarbon chain of 1 to 3 carbon atoms or an unsaturated hydrocarbon chain of 2 or 3 carbon atoms; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
or a salt, ether or ester thereof, provided that when R₁ and R₂ are each methyl or hydrogen, then R₃ may not be 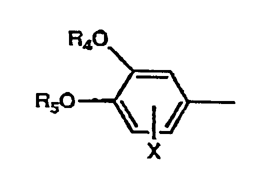
wherein R₄ and R₅ are independently hydrogen, alkyl, optionally substituted acyl or optionally substituted aroyl, lower alkyl sulfonyl or alkylcarbamoyl or taken together form a lower alkylidene or cycloalkylidene group and X is an electronegative group, for use as a cytoprotective agent.

Preferable salts of compounds of formula Ib are those formed with sodium, potassium, ammonium, calcium, magnesium or organic bases. Preferred ethers and esters of the compounds of formula Ib are acyl or aroyl derivatives which will hydrolyse under physiological conditions.

The compounds of formula I(b) may be prepared by reacting a compound of the formula II

R₁ - CO - CH₂ - CO - R₂ II

in which R₁ and R₂ are as defined above with a compound of the formula III

R₃ - Z III

in which R₃ is as defined above and Z is CHO or -CH₂-Q, wherein Q is halogen or some other activated group, in the presence of acid or base catalyst to produce a compound of formula I (a) 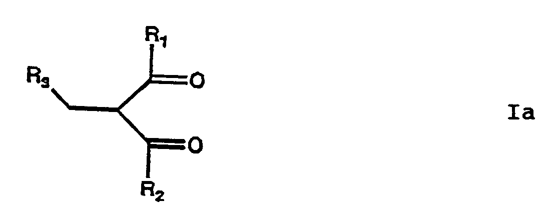
in which R₁, R₂ and R₃ are as defined above, whereafter the compound according to formula Ia is halogenated to give a compound of the formula IV 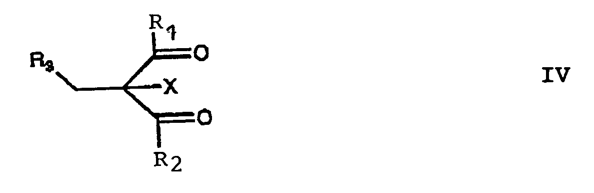
in which R₁, R₂ and R₃ are as defined above and X is halogen and dehydrohalogenating compound IV to produce a compound of the formula Ib.

The basic catalyst may be for example an organic base or an organic amine. The acid catalyst may be for example a mineral acid or sulfonic acid. The activated group Q may be a halogen or an alkyl or aryl sulphonate.

When halogenating a compound of formula Ia elementary halogen, preferably chlorine or bromine may be used, or another known halogenating agent such as sulfuryl chloride may be used.

The present invention provides a pharmaceutical composition comprising a compound of formula Ib as defined above or a salt, ether or ester thereof, and a pharmaceutically acceptable carrier or diluent.

The composition may be for example in the form of a tablet, dragee, capsule, suppository, emulsion, suspension or solution. It may contain a pharmaceutically acceptable additive or excipient such as a solvent, gel or dispersion forming agent, an antioxidant or a colourant.

The effective dose varies depending on whether the compounds are given for prophylaxis or for treatment of a condition which is already present. The daily dose and number of doses are dependent on the severity of the condition to be treated. The effective dose is generally from about 1 to 1000 mg of compound per day, preferably typically 100 to 600 mg per day.

The following examples illustrate the invention.

### Example 1

### 3-(4-carboxybenzylidene)-2,4-pentanedione

To a suspension containing 3.0g (0.02 mol) of 4-carboxybenxaldehyde and 3.0g (0.03 mol) of 2,4-pentanedione in 10 ml of 2-propanol was gradually added 3.0g (0.025 mol) of thionyl chloride with stirring and cooling (below 20°C). The mixture was stirred over night at 20°C. After filtering the product was washed with 2-propanol. Yield 1.8g,
mp 195-199°C.

### Example 2

### 3-(4-hydroxybenzylidene)-2,4-pentanedione

The procedure described in example 1 was repeated using 4-hydroxybenzaldehyde instead of 4-carboxybenzaldehyde.
Mp 123-127°C.

### Example 3

### 3-(4-methoxybenzylidene)-2,4-pentanedione

The procedure described in example 1 was repeated using 4-methoxybenzaldehyde instead of 4-carboxybenzaldehyde.
Mp 70-72°C.

### Example 4

### 3-(benzylidene)-2,4-pentanedione

The procedure described in example 1 was repeated using benzaldehyde instead of 4-carboxybenzaldehyde. Colourless oil, bp 186-188°C/16mm.

### Example 5

### 3-ethylidene-2,4-pentanedione

The procedure described in example 1 was repeated using acetaldehyde instead of 4-carboxybenzaldehyde. Colourless oil, bp 97°C/18mm.

### Example 6

### 1,4-bis-(2-acetyl-3-oxo-1-butenyl)benzene

To a mixture containing 1.34g of 1,4-benzenedicarboxaldehyde and 2.1g of 2,4-pentanedione in 20ml of 2-propanol was gradually added 3.0g of thionyl chloride with stirring and cooling below 20°C. The mixture was stirred over night at room temperature, filtered and washed with 2-propanol.
Yield 1.1g (55%).

### Example 7

### 3-acetyl-4-(2-thienyl)-3-buten-2-one

The procedure described in example 1 was repeated by using thiophene-2-carboxaldehyde instead of 4-carboxybenzaldehyde.
Yellowish oil, yield 70%.

### Example 8

### 3-acetyl-4-(2-furyl)-3-buten-2-one

A solution containing 2.9g of furfural, 5.0g of 2,4-pentanedione and 1g of ammonium acetate in 25 ml of 2-propanol was refluxed for 3 h. The solvent was evaporated in vacuo and the residue was purified using column chromatography. Yellowish oil, yield 61%.

### Example 9

### Dimethyl benzylidenemalonate

A mixture containing 10.6g of benzaldehyde, 13.2g of dimethylmalonate and 1ml of ethyl-di(2-propyl)amine was heated over night at 120°C. The mixture was distilled in vacuo and the fraction boiling at 170-180°C/18mm was collected. Colourless oil, yield 1.9g.

### Example 10

### 3-(3,4-dihydroxybenzylidene)-2,4-pentanedione

The process described in example 1 was repeated using 3,4-dihydroxybenzaldehyde instead of 4-carboxybenzaldehyde
Mp 134-137°C.

### Example 11

### 3-benzylidene-2,4-pentandione

To a solution containing 6.3g of 3-benzyl-2,4-pentanedione in 50ml of dichloromethane was gradually added 5.3g of bromine in 20ml of dichloromethane under cooling (0-5°C). The solution was stirred for 10 min. at 0°C and the solvent evaporated in vacuo. The residue was dissolved in 100ml of pyridine and relfuxed for 30 min. Pyridine was evaporated in vacuo, the residue was dissolved in dichloromethane and washed first with 6M hydrochloric acid and then with 2.6M NaOH. The solvent was evaporated and the residue distilled in vacuo. Bp 186-188°C/16mm, yield 1.2g.

### Example 12

### 3-(2-Trifluoromethylbenzylidene)-2,4-pentanedione

Thionylchloride (4 ml) and a catalytic amount of water (0.05 ml) were added at room temperature to a solution of 2-trifluoromethylbenzaldehyde (8.7 g) and 2,4-pentanedione (5.01 g) in trifluoroacetic acid (10 ml). The solution was stirred over night at 20°C. The solvent was evaporated and the residue was distilled in vacuo, bp 110°C/1.5 mbar.
Yield 5.3 g (41%).

### Example 13

### 3-(4-Trifluoromethylbenzylidene)-2,4-pentanedione

4-Trifluoromethylbenzaldehyde (8.7 g) was condensed with 2,4-pentanedione (5.01 g) in a similar manner as described above. The crude product was crystallized from a mixture of ether-petroleum ether (1:1), mp 46-48°C, yield 3.8 g (30%).

### Example 14

### 3-(3-Cyanobenzylidene)-2,4-pentanedione

3-cyanobenzaldehyde (2.62 g) was condensed with 2,4-pentanedione (3.0 g) in 2-propanol (10 ml) in the presence of ammonium acetate. Mp 63-64°C, yield 1.27 g (30%).

### Example 15

### 3-(4-Cyanobenzylidene)-2,4-pentanedione

4-Cyanobenzaldehyde (2.62 g) was condensed with 2,4-pentanedione (3.0 g) in 2-propanol (10 ml) in the presence of ammonium acetate. Mp 86-88°C, yield 0.55 g (13%).

### Example 16

### 3-(4-Methoxybenzylidene)-2,4-pentanedione

4-Methoxybenzyl chloride was condensed with 2,4-pentanedione as described in Example 6 to give 3-(4-methoxybenzyl)-2,4-pentanedione as a yellow oil. The crude product was treated first with bromine and then with pyridine as described in Example 12 to give the title compound, Mp 71-72°C.

### Example 17

### 3-(4-Trifluoromethylbenzylidene)-2,4-pentanedione

The procedure described in Example 17 was repeated by using 4-trifluoromethylbenzyl chloride. Mp 46-47°C.

### Example 18

### 3-(4-Cyanobenzylidene)-2,4-pentanedione

The procedure described in Example 17 was repeated by using 4-cyanobenzyl chloride. Mp 86-88°C.

### Example 19

### 3-Acetyl-4-(2-thienyl)-3-buten-2-one

The procedure described in Example 17 was repeated by using 2-chloromethylthiophene. Yellow oil.

### Example 20

### 3-Acetyl-4-(2-furyl)-3-buten-2-one

The procedure described in Example 17 was repeated by using 2-chloromethylfuran. Yellow oil.

### Example 21

### 3-(4-Carboxybenzylidene)-2,4-pentanedione

The procedure described in Example 17 was repeated by using methyl 4-chloromethylbenzoate to give 3-(4-methoxycarbonylbenzylidene)-2,4-pentanedione as a yellow oil. The crude product was hydrolyzed with dilute sodium hydroxide solution and acidified with hydrochloric acid to give the title compound, Mp 196-199°C.

### Effect of substituted β-diketones in vivo

Oral administration of absolute ethanol to rats results in severe gastric damage consisting of grossly hemorrhagic and necrotic lesions. Compounds which are able to prevent the ethanol-induced lesions are called cytoprotective or gastroprotective agents.

Male Wistar rates were orally dosed (5 ml/kg) with a test compound suspended in 5% gum arabic. Control animals received pure vehicle. Half an hour later the rats were orally administered with 1ml of absolute ethanol. The animals were sacrificed one hour after ethanol administration and the total area of macroscopic lesions in each stomach was calculated in mm². The extent of duodenal damage was measured in cm from pylorus.

The results are summarised in Table 1.

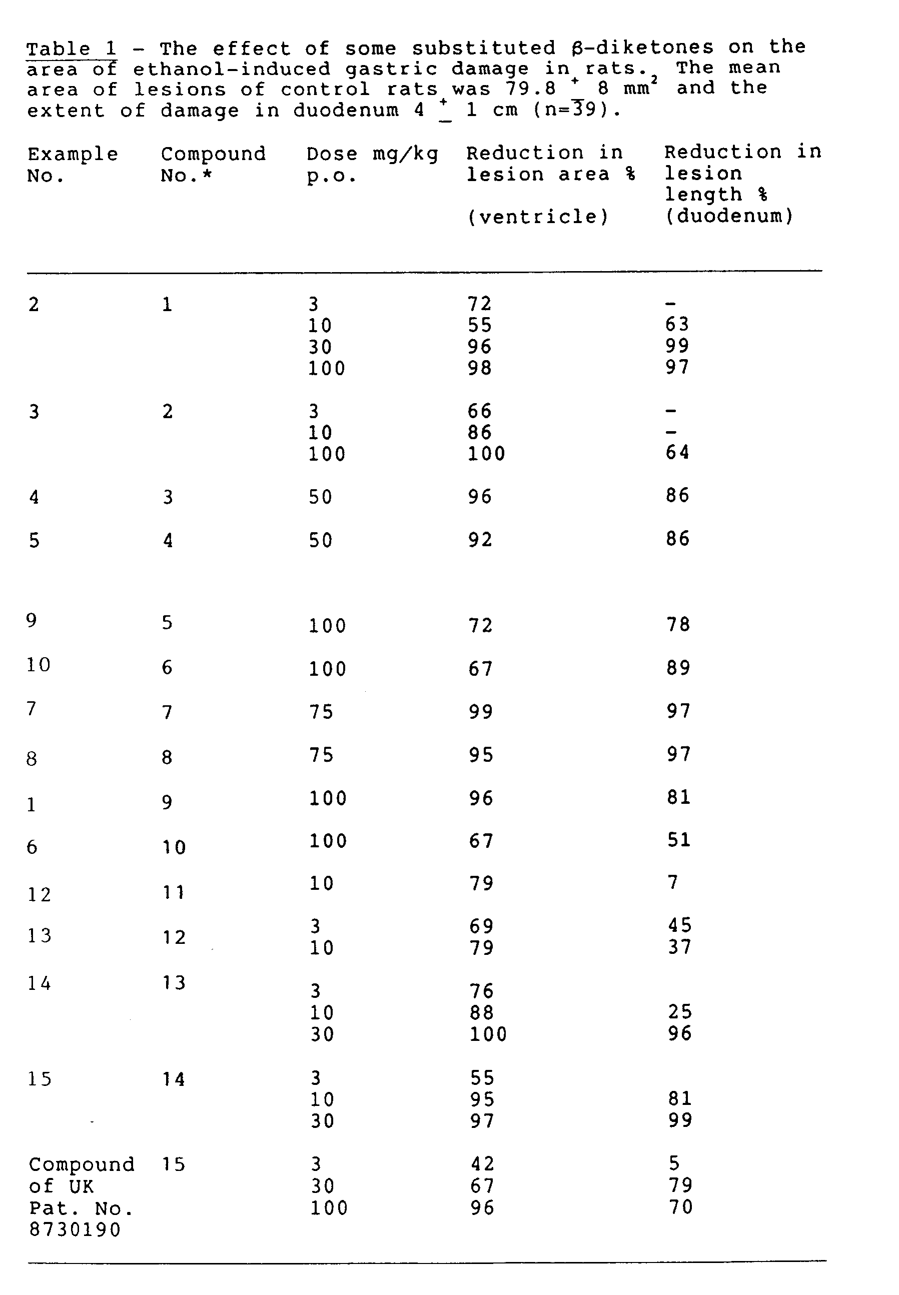

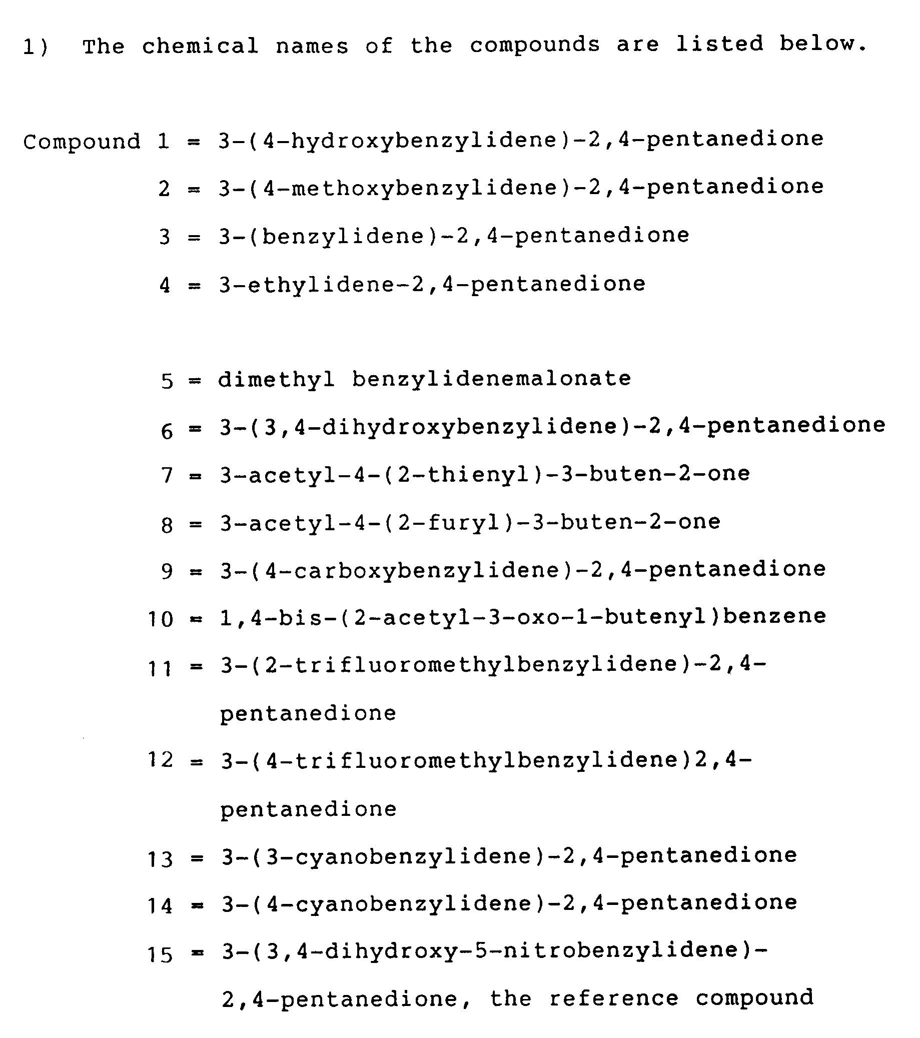

All the substituted β-diketones significantly reduce the ethanol-induced mucosal lesions in the stomach, and this reduction is dependant on the dose administered. Moreover, the damaged area of the duodenum was smaller and less severe in animals treated with the present compounds than in the control animals. It can therefore be seen that the present compounds have gastroprotective activity both in the stomach and in the duodenum.

## Claims (Claims for the following Contracting State(s): AT, BE, CH, DE, FR, GB, IT, LI, LU, NL, SE)

1. A compound which is of the formula Ib: 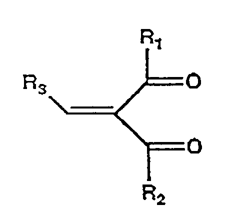 wherein R₁ and R₂ are independently C₁₋₆ alkyl which is unsubstituted or substituted by hydroxy, C₁₋₆ alkoxy, halogeno, nitro or amino; C₁₋₆ alkoxy; phenyl or naphthyl which is unsubstituted or substituted by C₁₋₆ alkyl, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno nitro or amino;
R₃ is C₁₋₆ alkyl which is substituted by hydroxy, C₁₋₆ alkoxy, halogeno, nitro or amino; phenyl or naphthyl which are substituted by trifluoromethyl or cyano; or one of the following heteroaryl groups: quinolyl, thienyl or furyl which are substituted by C₁₋₆ alkyl, hydroxy, C₁₋₆ alkoxy, halogeno, nitro or amino;
or a salt, ether or ester thereof, provided that when R₃ is substituted furyl it is not substituted by alky, halogeno or nitro and if R₃ is a phenyl which is substituted by cyano then the cyano is not in the para position.

2. A compound according to claim 1 in which R₁ and R₂ are each C₁₋₄ alkyl.

3. 3-(4-trifluoromethylbenzylidene)-2,4-pentanedione.

4. 3-(3-cyanobenzylidene)-2,4-pentanedione.

5. A process for the preparation of a compound Ib according to claim 1, comprising reacting a compound having formula II
R₁ - CO - CH₂ - CO - R₂ II
in which R₁ and R₂ are as defined in claim 1, with a compound having formula III
R₃Z III
in which R₃ is as defined in claim 1, Z is CHO or -CH₂-Q and Q is halogen or an other activated group, in the presence of acid or basic catalyst to produce the compound Ia 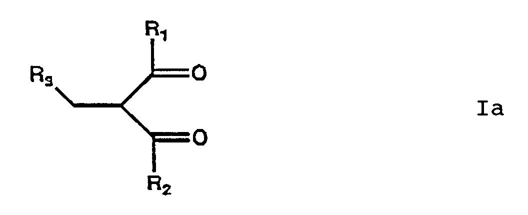 in which R₁, R₂ and R₃ are as defined in claim 1, whereafter the compound of formula Ia is halogenated to give a compound of formula IV 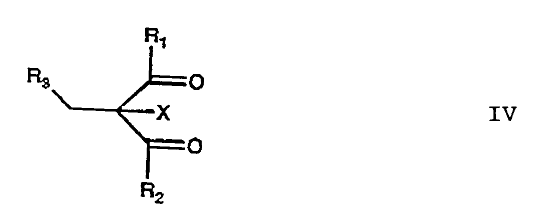 in which R₁, R₂ and R₃ are as defined in claim 1 and X is halogen and dehydrohalogenating compound IV to produce a compound of formula Ib.

6. A process according to claim 5 in which the basic catalyst is an inorganic base or an organic amine.

7. A process according to claim 5 in which the acid catalyst is a mineral acid or a sulfonic acid.

8. A process according to claim 5, 6 or 7 in which the halogenating agent is chlorine, bromine or sulfuryl chloride.

9. A compound of formula 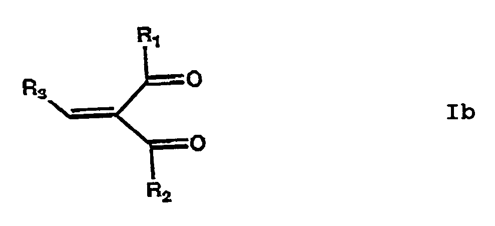 wherein R₁ and R₂ are independently C₁₋₆ alkyl which is unsubstituted or substituted by hydroxy, C₁₋₆ alkoxy, halogeno, nitro or amino; C₁₋₆ alkoxy; phenyl or naphthyl which is unsubstituted or substituted by C₁₋₆ alkyl, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
R₃ is C₁₋₆ alkyl which is unsubstituted or substituted by hydroxy, C₁₋₆ alkoxy, halogeno, nitro or amino; phenyl or naphthyl which are unsubstituted or substituted by C₁₋₆ alkyl, C₁₋₆ alkoxy, trifluoromethyl, cyano, amino or carboxy or a diketo group attached to the aryl group directly or via a saturated hydrocarbon chain of 1 to 3 carbon atoms or an unsaturated hydrocarbon chain of 2 or 3 carbon atoms; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
or a salt, ether or ester thereof, provided that when R₁ and R₂ are each methyl or hydrogen, then R₃ may not be 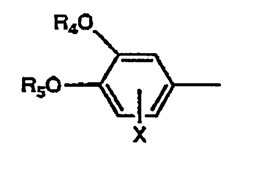 wherein R₄ and R₅ are independently hydrogen, alkyl, optionally substituted acyl or optionally substituted aroyl, lower alkyl sulfonyl or alkylcarbamoyl or taken together form a lower alkylidene or cycloalkylidene group and X is an electronegative group, for use as a pharmaceutical.

10. A compound according to claim 9 in which R₁ and R₂ are C₁₋₄ alkyl and R₃ is phenyl which is unsubstituted or substituted by C₁₋₄ alkoxy, carboxy, trifluoromethyl or cyano, or is thienyl or furyl which is unsubstituted or substituted by C₁₋₄ alkoxy, trifluoromethyl or cyano.

11. A compound according to claims 9 or 10 for use in the treatment or prophylaxis of ulcers or lesions in the gastrointestinal tract.

12. 3-(4-methoxybenzylidene)-2,4-pentanedione for use in the treatment or prophylaxis of ulcers or lesions in the gastrointestinal tract.

13. 3-(benzylidene)-2,4-pentanedione for use in the treatment or prophylaxis of ulcers or lesions in the gastrointestinal tract.

14. 3-ethylidene-2,4-pentanedione for use in the treatment or prophylaxis of ulcers or lesions in the gastrointestinal tract.

15. Dimethylbenzylidenemalonate for use in the treatment or prophylaxis of ulcers or lesions in the gastrointestinal tract.

16. 1,4-bis(2-acetyl-3-oxo-1-butenyl)benzene for use in the treatment or prophylaxis of ulcers or lesions in the gastrointestinal tract.

17. 3-acetyl-4-(2-thienyl)-3-buten-2-one for use in the treatment or prophylaxis of ulcers or lesions in the gastrointestinal tract.

18. 3-acetyl-4-(2-furyl)-3-buten-2-one for use in the treatment or prophylaxis of ulcers or lesions in the gastrointestinal tract.

19. 3-(4-cyanobenzylidene)-2,4-pentanedione for use in the treatment or prophylaxis of ulcers or lesions in the gastrointestinal tract.

20. 3-(4-carboxybenzylidene)-2,4-pentanedione for use in the treatment or prophylaxis of ulcers or lesions in the gastrointestinal tract.

21. 3-(2-trifluoromethylbenzylidene)-2,4-pentanedione for use in the treatment or prophylaxis of ulcers or lesions in the gastrointestinal tract.

22. 3-(3-cyanobenzylidene)-2,4-pentanedione for use in the treatment or prophylaxis of ulcers or lesions in the gastrointestinal tract.

23. Use of a compound as defined in any one of claims 9 to 22 for the manufacture of a medicament for the treatment or prophylaxis of ulcers or lesions in the gastrointestinal tract.

24. Use of 3-benzylidene-2,4-pentanedione for the manufacture of a medicament for the treatment or prophylaxis of ulcers or lesions in the gastrointestinal tract.

25. A pharmaceutical composition comprising a compound according to any one of claims 1 to 4 or as defined in any one of claims 9 to 22 and a pharmaceutically acceptable carrier or diluent.

26. A pharmaceutical composition comprising 3-(3-cyanobenzylidene)-2,4-pentanedione and a pharmaceutically acceptable carrier or diluent.

## Claims (Claims for the following Contracting State(s): ES, GR)

1. A process for the preparation of a compound of formula Ib: 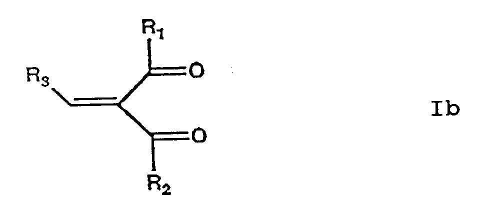 wherein R₁ and R₂ are independently C₁₋₆ alkyl which is unsubstituted or substituted by hydroxy, C₁₋₆ alkoxy, halogeno, nitro or amino; C₁₋₆alkoxy; phenyl or naphthyl which is unsubstituted or substituted by C₁₋₆ alkyl, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
R₃ is C₁₋₆ alkyl which is substituted by hydroxy, C₁₋₆ alkoxy, halogeno, nitro or amino; phenyl or naphthyl which are substituted by trifluoromethyl or cyano; or one of the following heteroaryl groups: quinolyl, thienyl or furyl which are substituted by C₁₋₆ alkyl, hydroxy, C₁₋₆ alkoxy, halogeno, nitro or amino;
or a salt, ether or ester thereof, provided that when R₃ is substituted furyl it is not substituted by alkyl, halogeno or nitro and if R₃ is a phenyl which is substituted by cyano then the cyano is not in the para position, comprising reacting a compound having formula II
R₁ - CO - CH₂ - CO - R₂ II
in which R₁ and R₂ are as defined above, with a compound having formula III
R₃Z III
in which R₃ is as defined above, Z is CHO or -CH₂-Q and Q is halogen or another activated group, in the presence of acid or basic catalyst to produce the compound Ia 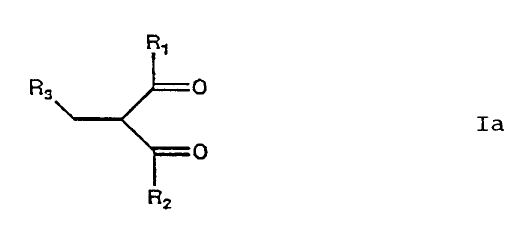 in which R₁, R₂ and R₃ are as defined above, whereafter the compound of formula Ia is halogenated to give a compound of formula IV 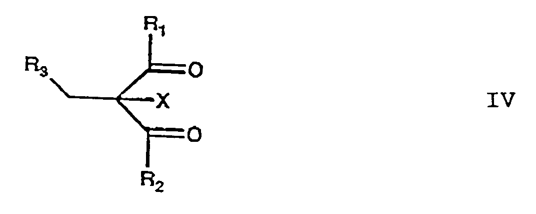 in which R₁, R₂ and R₃ are as defined above and X is halogen and dehydrohalogenating compound IV to produce a compound of formula Ib.

2. A process according to claim 1 in which the basic catalyst is an inorganic base or an organic amine.

3. A process according to claim 1 in which the acid catalyst is a mineral acid or a sulfonic acid.

4. A process according to claim 1, 2 or 3 in which the halogenating agent is chlorine, bromine or sulfuryl chloride.

5. A process according to any one of the preceding claims for the preparation of a compound in which R₁ and R₂ are each C₁₋₄ alkyl.

6. A process according to any one of claims 1 to 4 for the preparation of 3-(2-trifluoromethylbenzylidene)-2,4-pentanedione.

7. A process according to any one of claims 1 to 4 for the preparation of 3-(4-trifluoromethylbenzylidene)-2,4-pentanedione.

8. A process according to any one of claims 1 to 4 for the preparation of 3-(3-cyanobenzylidene)-2,4-pentanedione.

9. A process for the preparation of a pharmaceutical composition comprising mixing a compound of formula 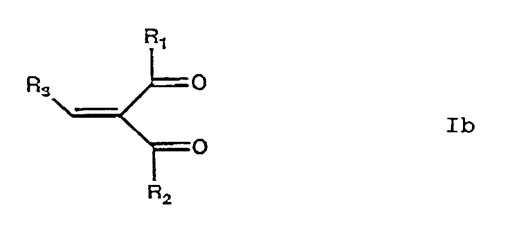 wherein R₁ and R₂ are independently C₁₋₆ alkyl which is unsubstituted or substituted by hydroxy, C₁₋₆ alkoxy, halogeno, nitro or amino; C₁₋₆ alkoxy; phenyl or naphthyl which is unsubstituted or substituted by C₁₋₆ alkoxy, hydroxy, C₁₋₆ alkoxy, amino or carboxy; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
R₃ is C₁₋₆ alkyl which is unsubstituted or substituted by hydroxy, C₁₋₆ alkoxy, halogeno, nitro or amino; phenyl or naphthyl, which are unsubstituted or substituted by C₁₋₆ alkyl, C₁₋₆ alkoxy, trifluoromethyl, cyano, amino or carboxy or a diketo group attached to the aryl group directly or via a saturated hydrocarbon chain of 1 to 3 carbon atoms or an unsaturated hydrocarbon chain of 2 or 3 carbon atoms; or one of the following heteroaryl groups: pyridyl, quinolyl, thienyl or furyl which are unsubstituted or substituted by C₁₋₆ alkyl, hydroxy, C₁₋₆ alkoxy, halogeno, nitro or amino;
or a salt, ether or ester thereof, provided that when R₁ and R₂ are each methyl or hydrogen, then R₃ may not be 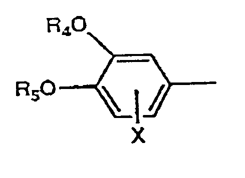 wherein R₄ and R₅ are independently hydrogen, alkyl, optionally substituted acyl or optionally substituted aroyl, lower alkyl sulfonyl or alkylcarbamoyl or taken together form a lower alkylidene or cycloalkylidene group and X is an electronegative group, with a pharmaceutically acceptable carrier or diluent.

10. A process according to claim 9 wherein the compound of formula Ib is a compound wherein R₁ and R₂ are C₁₋₄ alkyl and R₃ is phenyl which is unsubstituted or substituted by C₁₋₄ alkoxy, carboxy, trifluoromethyl or cyano, or is thienyl or furyl which is unsubstituted or substituted by C₁₋₄ alkoxy, trifluoromethyl or cyano.

11. A process according to claim 9 wherein the compound of formula Ib is 3-(4-methoxybenzylidene)-2,4-pentanedione.

12. A process according to claim 9 wherein the compound of formula Ib is 3-(benzylidene)-2,4-pentanedione.

13. A process according to claim 9 wherein the compound of formula Ib is 3-ethylidene-2,4-pentanedione.

14. A process according to claim 9 wherein the compound of formula Ib is dimethylbenzylidenemalonate.

15. A process according to claim 9 wherein the compound of formula Ib is 1,4-bis(2-acetyl-3-oxo-1-butenyl) benzene.

16. A process according to claim 9 wherein the compound of formula Ib is 3-acetyl-4-(2-thienyl)-3-buten-2-one.

17. A process according to claim 9 wherein the compound of formula Ib is 3-acetyl-4-(2-furyl)-3-buten-2-one.

18. A process according to claim 9 wherein the compound of formula Ib is 3-(4-cyanobenzylidene)-2,4-pentanedione.

19. A process according to claim 9 wherein the compound of formula Ib is 3-(4-carboxybenzylidene)-2,4-pentanedione.

20. A process according to claim 9 or 10 wherein the compound of formula Ib is a compound as defined in any one of claims 1 and 5 to 8.

21. A process according to claim 9 wherein the compound of formula Ib is 3-(3-cyanobenzylidene)-2,4-pentanedione.

22. Use of a compound as defined in any one of claims 6 and 9 to 19 for the manufacture of a medicament for the treatment or prophylaxis of ulcers or lesions in the gastrointestinal tract.

23. Use according to claim 22 wherein the compound is 3-(3-cyanobenzylidene)-2,4-pentanedione.

## Patentansprüche (Patentansprüche für folgende(n) Vertragsstaat(en): AT, BE, CH, DE, FR, GB, IT, LI, LU, NL, SE)

1. Verbindung der Formel Ib: 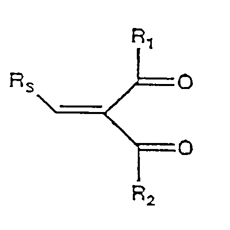 worin R₁ und R₂ unabhängig voneinander ein nicht substituiertes oder ein mit Hydroxy, C₁₋₆-Alkoxy, Halogen, Nitro oder Amino subsituiertes C₁₋₆-Alkyl; ein C₁₋₆-Alkoxy; Phenyl oder Naphthyl das nicht substituiert oder mit C₁₋₆-Alkyl, Hydroxy, C₁₋₆-Alkoxy, Amino oder Carboxy substituiert ist; oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amino substituiert sind, bedeutet;
R₃ ein mit Hydroxy, C₁₋₆-Alkoxy, Halogen, Nitro oder Amino substituiertes C₁₋₆-Alkyl; ein mit Trifluormethyl oder Cyano substituiertes Phenyl oder Naphthyl; oder eine der folgenden Heteroarylgruppen; Chinolyl, Thienyl oder Furyl, die mit C₁₋₆-Alkyl, Hydroxy, C₁₋₆-Alkoxy, Halogen, Nitro oder Amino substituiert sind, bedeutet;
oder ein Salz, Ether oder Ester von diesen,
unter der Voraussetzung, daß - wenn R₃ ein substituiertes Furyl ist - es nicht mit Alkyl, Halogen oder Nitro substituiert ist, und das Cyano nicht in para-Stellung steht, wenn R₃ ein Cyano-substituiertes Phenyl ist.

2. Verbindung nach Anspruch 1, worin R₁ und R₂ jeweils ein C₁₋₄-Alkyl bedeutet.

3. 3-(4-Trifluormethylbenzyliden)-2,4-pentandion.

4. 3-(3-Cyanobenzyliden)-2,4-pentandion.

5. Verfahren zur Herstellung einer Verbindung Ib nach Anspruch 1, welches umfaßt: das Umsetzen einer Verbindung der Formel II
R₁ -CO - CH₂ - CO - R₂ II
worin R₁ und R₂ die in Anspruch 1 definierte Bedeutung haben, mit einer Verbindung der Formel III
R₃Z III
worin R₃ die in Anspruch 1 definierte Bedeutung hat, Z = CHO oder -CH₂-Q ist und Q Halogen oder eine andere aktivierte Gruppe ist,
in Anwesenheit eines sauren oder basischen Katalysators zur Herstellung der Verbindung Ia 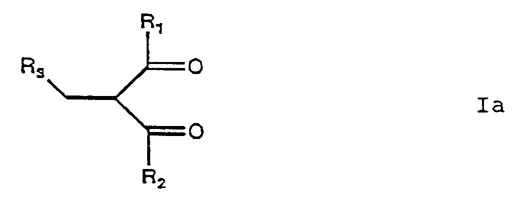 worin R₁, R₂ und R₃ die in Anspruch 1 definierten Bedeutungen haben,
wonach die Verbindung der Formel Ia halogeniert wird, um eine Verbindung der Formel IV zu ergeben 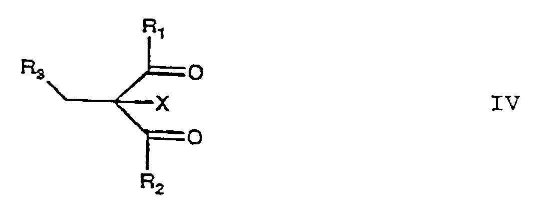 worin R₁, R₂ und R₃ die in Anspruch 1 definierten Bedeutungen haben, und X Halogen ist,
und Dehydrohalogenierung der Verbindung IV zum Erhalt einer Verbindung der Formel Ib.

6. Verfahren nach Anspruch 5, worin der basische Katalysator eine anorganische Base oder ein organisches Amin ist.

7. Verfahren nach Anspruch 5, worin der Säurekatalysator eine Mineralsäure oder eine Sulfonsäure ist.

8. Verfahren nach Anspruch 5, 6 oder 7, worin das Halogenierungsmittel Chlor, Brom oder Sulfurylchlorid ist.

9. Verbindung der Formel 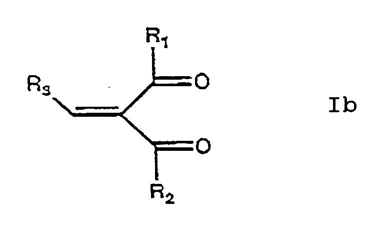 worin R₁ und R₂ unabhängig voneinander ein nicht substituiertes oder mit Hydroxy, C₁₋₆-Alkoxy, Halogen, Nitro oder Aminosubstituiertes C₁₋₆-Alkyl; ein C₁₋₆-Alkoxy; Phenyl oder Naphthyl, das nicht substituiert oder mit C₁₋₆-Alkyl, Hydroxy, C₁₋₆Alkoxy, Amino oder Carboxy substituiert ist; oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amino substituiert sind, bedeuten;
R₃ bedeutet: ein C₁₋₆-Alkyl, das nicht substituiert oder mit Hydroxy, C₁₋₆-Alkoxy, Halogen, Nitro oder Amino substituiert ist; Phenyl oder Naphthyl, die nicht substituiert oder mit C₁₋₆-Alkyl, C₁₋₆-Alkoxy, Trifluormethyl, Cyano, Amino oder Carboxy substituiert sind, oder eine Diketo-Gruppe, die direkt oder über eine gesättigte Kohlenwasserstoffkette mit 1-3 Kohlenstoffatomen oder eine ungesättigte Kohlenwasserstoffkette mit 2 oder 3 Kohlenstoffatomen an die Arylgruppe gebunden ist; oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amin substituiert sind;
oder ein Salz, Ether oder Ester von diesen,
unter der Voraussetzung, daß - wenn R₁ und R₂ jeweils Methyl oder Wasserstoff sind - R₃ nicht 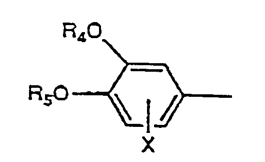 sein kann,
worin R₄ und R₅ unabhängig voneinander Wasserstoff, Alkyl, gegebenenfalls substituiertes Acyl oder gegebenenfalls substituiertes Aroyl, ein niederes Alkylsulfonyl oder Alkylcarbamoyl sind, oder zusammen eine niedere Alkyliden- oder Cycloalkylidengruppe bilden, und X eine elektronegative Gruppe ist,
zur Verwendung als ein Pharmazeutikum.

10. Verbindung nach Anspruch 9, worin R₁ und R₂ ein C₁₋₄-Alkyl und R₃ Phenyl ist, das nicht substituiert oder mit C₁₋₄-Alkoxy, Carboxy, Trifluormethyl oder Cyano substituiert ist, oder Thienyl oder Furyl ist, das nicht substituiert oder mit C₁₋₄-Alkoxy, Trifluormethyl oder Cyano substituiert ist.

11. Verbindung nach Anspruch 9 oder 10 zur Verwendung bei der Behandlung oder Prophylaxe von Geschwüren oder Läsionen im gastrointestinalen Trakt.

12. 3-(4-Methoxybenzyliden)-2,4-pentandion zur Verwendung bei der Behandlung oder Prophylaxe von Geschwüren oder Läsionen im gastrointestinalen Trakt.

13. 3-(Benzyliden)-2,4-pentandion zur Verwendung bei der Behandlung oder Prophylaxe von Geschwüren oder Läsionen im gastrointestinalen Trakt.

14. 3-Ethyliden-2,4-pentandion zur Verwendung bei der Behandlung oder Prophylaxe von Geschwüren oder Läsionen im gastrointestinalen Trakt.

15. Dimethylbenzylidenmalonat zur Verwendung bei der Behandlung oder Prophylaxe von Geschwüren oder Läsionen im gastrointestinalen Trakt.

16. 1,4-Bis(2-acetyl-3-oxo-1-butenyl)benzol zur Verwendung bei der Behandlung oder Prophylaxe von Geschwüren oder Läsionen im gastrointestinalen Trakt.

17. 3-Acetyl-4-(2-thienyl)-3-buten-2-on zur Verwendung bei der Behandlung oder Prophylaxe von Geschwüren oder Läsionen im gastrointestinalen Trakt.

18. 3-Acetyl-4-(2-furyl)-3-buten-2-on zur Verwendung bei der Behandlung oder Prophylaxe von Geschwüren oder Läsionen im gastrointestinalen Trakt.

19. 3-(4-Cyanobenzyliden)-2,4-pentandion zur Verwendung bei der Behandlung oder Prophylaxe von Geschwüren oder Läsionen im gastrointestinalen Trakt.

20. 3-(4-Carboxybenzyliden)-2,4-pentandion zur Verwendung bei der Behandlung oder Prophylaxe von Geschwüren oder Läsionen im gastrointestinalen Trakt.

21. 3-(2-Trifluormethylbenzyliden)-2,4-pentandion zur Verwendung bei der Behandlung oder Prophylaxe von Geschwüren oder Läsionen im gastrointestinalen Trakt.

22. 3-(3-Cyanobenzyliden)-2,4-pentandion zur Verwendung bei der Behandlung oder Prophylaxe von Geschwüren oder Läsionen im gastrointestinalen Trakt.

23. Verwendung einer Verbindung nach einem der Ansprüche 9 bis 22 zur Herstellung eines Medikaments zur Behandlung oder Prophylaxe von Geschwüren oder Läsionen im gastrointestinalen Trakt.

24. Verwendung von 3-Benzyliden-2,4-pentandion zur Herstellung eines Medikaments zur Behandlung oder Prophylaxe von Geschwüren oder Läsionen im gastrointestinalen Trakt.

25. Pharmazeutische Zusammensetzung, die eine Verbindung nach einem der Ansprüche 1 bis 4 oder nach einem der Ansprüche 9 bis 22, mit einem pharmazeutisch geeigneten Träger oder Verdünnungsmittel umfaßt.

26. Pharmazeutische Zusammensetzung, die 3-(3-Cyanobenzyliden)-2,4-pentandion und einen pharmazeutisch geeigneten Träger oder Verdünnungsmittel umfaßt.

## Patentansprüche (Patentansprüche für folgende(n) Vertragsstaat(en): ES, GR)

1. Verfahren zur Herstellung einer Verbindung der Formel Ib: 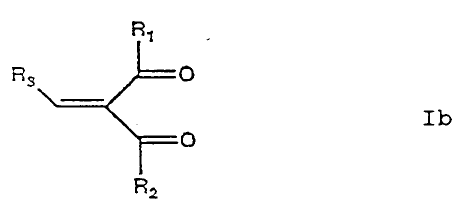 worin R₁ und R₂ unabhängig voneinander ein nicht substituiertes oder ein mit Hydroxy, C₁₋₆-Alkoxy, Halogen, Nitro oder Amino subsituiertes C₁₋₆-Alkyl; ein C₁₋₆-Alkoxy; Phenyl oder Naphthyl; das nicht substituiert oder mit C₁₋₆-Alkyl, Hydroxy, C₁₋₆-Alkoxy, Amino oder Carboxy substituiert ist oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amino substituiert sind, bedeutet;
R₃ ein mit Hydroxy, C₁₋₆-Alkoxy, Halogen, Nitro oder Amino substituiertes C₁₋₆-Alkyl; ein mit Trifluormethyl oder Cyano substituiertes Phenyl oder Naphthyl; oder eine der folgenden Heteroarylgruppen; Chinolyl, Thienyl oder Furyl, die mit C₁₋₆-Alkyl, Hydroxy, C₁₋₆-Alkoxy, Halogen, Nitro oder Amino substituiert sind, bedeutet;
oder ein Salz, Ether oder Ester von diesen,
unter der Voraussetzung, daß - wenn R₃ ein substituiertes Furyl ist - es nicht mit Alkyl, Halogen oder Nitro substituiert ist, und das Cyano nicht in para-Stellung steht, wenn R₃ ein Cyano-substituiertes Phenyl ist,
welches umfaßt: das Umsetzen einer Verbindung der Formel II
R₁ -CO - CH₂ - CO - R₂ II
worin R₁ und R₂ die oben definierte Bedeutung haben, mit einer Verbindung der Formel III
R₃Z III
worin R₃ die oben definierte Bedeutung hat, Z = CHO oder -CH₂-Q ist und Q Halogen oder eine andere aktivierte Gruppe ist,
in Anwesenheit eines sauren oder basischen Katalysators zur Herstellung der Verbindung Ia 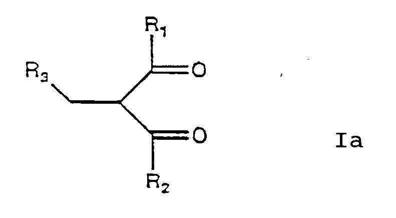 worin R₁, R₂ und R₃ die in Anspruch 1 definierten Bedeutungen haben,
wonach die Verbindung der Formel Ia halogeniert wird, um eine Verbindung der Formel IV 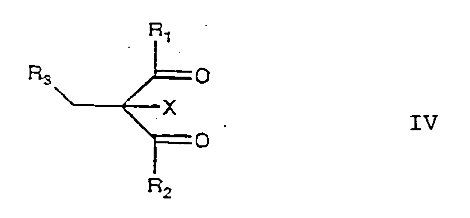 zu ergeben,
worin R₁, R₂ und R₃ die oben definierten Bedeutungen haben, und X Halogen ist,
und Dehydrohalogenierung der Verbindung IV zum Erhalt einer Verbindung der Formel Ib.

2. Verfahren nach Anspruch 1, worin der basische Katalysator eine anorganische Base oder ein organisches Amin ist.

3. Verfahren nach Anspruch 1, worin der Säurekatalysator eine Mineralsäure oder eine Sulfonsäure ist.

4. Verfahren nach Anspruch 1, 2 oder 3, worin das Halogenierungsmittel Chlor, Brom oder Sulfurylchlorid ist.

5. Verfahren nach einem der vorhergehenden Ansprüche zur Herstellung einer Verbindung, worin R₁ und R₂ jeweils C₁₋₄-Alkyl ist.

6. Verfahren nach einem der Ansprüche 1 bis 4 zur Herstellung von 3-(2-Trifluormethylbenzyliden)-2,4-pentandion.

7. Verfahren nach einem der Ansprüche 1 bis 4 zur Herstellung von 3-(4-Trifluormethylbenzyliden)-2,4-pentandion.

8. Verfahren nach einem der Ansprüche 1 bis 4 zur Herstellung von 3-(3-Cyanobenzyliden)-2,4-pentandion.

9. Verfahren zur Herstellung einer pharmazeutischen Zusammensetzung, welches umfaßt: das Mischen einer Verbindung der Formel 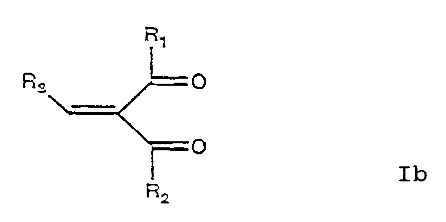 worin worin R₁ und R₂ unabhängig voneinander ein nicht substituiertes oder mit Hydroxy, C₁₋₆-Alkoxy, Halogen, Nitro oder Amino-substituiertes C₁₋₆-Alkyl; ein C₁₋₆-Alkoxy; Phenyl oder Naphthyl, das nicht substituiert oder mit C₁₋₆-Alkyl, Hydroxy, C₁₋₆Alkoxy, Amino oder Carboxy substituiert ist; oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amino substituiert sind, bedeuten;
R₃ bedeutet: ein C₁₋₆-Alkyl, das nicht substituiert oder mit Hydroxy, C₁₋₆-Alkoxy, Halogen, Nitro oder Amino substituiert ist; Phenyl oder Naphthyl, die nicht substituiert oder mit C₁₋₆-Alkyl, C₁₋₆-Alkoxy, Trifluormethyl, Cyano, Amino oder Carboxy substituiert sind, oder eine Diketo-Gruppe, die direkt oder über eine gesättigte Kohlenwasserstoffkette mit 1-3 Kohlenstoffatomen oder eine ungesättigte Kohlenwasserstoffkette mit 2 oder 3 Kohlenstoffatomen an die Arylgruppe gebunden ist; oder eine der folgenden Heteroarylgruppen: Pyridyl, Chinolyl, Thienyl oder Furyl, die nicht substituiert oder mit C₁₋₆-Alkyl, Hydroxy, C₁₋₆-Alkoxy, Halogen, Nitro oder Amino substituiert sind;
oder ein Salz, Ether oder Ester von diesen,
unter der Voraussetzung, daß - wenn R₁ und R₂ jeweils Methyl oder Wasserstoff sind - R₃ nicht 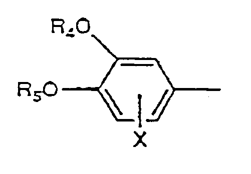 sein kann,
worin R₄ und R₅ unabhängig voneinander Wasserstoff, Alkyl, gegebenenfalls substituiertes Acyl oder gegebenenfalls substituiertes Aroyl, ein niederes Alkylsulfonyl oder Alkylcarbamoyl sind, oder zusammen eine niedere Alkyliden- oder Cycloalkylidengruppe bilden, und X eine elektronegative Gruppe ist, mit einem pharmazeutisch geeigneten Träger oder Verdünnungsmittel.

10. Verfahren nach Anspruch 9, worin die Verbindung der Formel Ib eine Verbindung ist, worin R₁ und R₂ ein C₁₋₄-Alkyl und R₃ Phenyl ist, das nicht substituiert oder mit C₁₋₄-Alkoxy, Carboxy, Trifluormethyl oder Cyano substituiert ist, oder Thienyl oder Furyl ist, das nicht substituiert oder mit C₁₋₄-Alkoxy, Trifluormethyl oder Cyano substituiert ist.

11. Verfahren nach Anspruch 9, worin die Verbindung der Formel Ib 3-(4-Methoxybenzyliden)-2,4-pentandion ist.

12. Verfahren nach Anspruch 9, worin die Verbindung der Formel Ib 3-(Benzyliden)-2,4-pentandion ist.

13. Verfahren nach Anspruch 9, worin die Verbindung der Formel Ib 3-Ethyliden-2,4-pentandion ist.

14. Verfahren nach Anspruch 9, worin die Verbindung der Formel Ib Dimethylbenzylidenmalonat ist.

15. Verfahren nach Anspruch 9, worin die Verbindung der Formel Ib 1,4-Bis(2-acetyl-3-oxo-1-butenyl)benzen ist.

16. Verfahren nach Anspruch 9, worin die Verbindung der Formel Ib 3-Acetyl-4-(2-thienyl)-3-buten-2-on ist.

17. Verfahren nach Anspruch 9, worin die Verbindung der Formel Ib 3-Acetyl-4-(2-furyl)-3-buten-2-on ist.

18. Verfahren nach Anspruch 9, worin die Verbindung der Formel Ib 3-(4-Cyanobenzyliden)-2,4-pentandion ist.

19. Verfahren nach Anspruch 9, worin die Verbindung der Formel Ib 3-(4-Carboxybenzyliden)-2,4-pentandion ist.

20. Verfahren nach Anspruch 9, worin die Verbindung der Formel Ib eine Verbindung ist, wie sie in einem der Ansprüche 1 und 5 bis 8 definiert worden ist.

21. Verfahren nach Anspruch 9, worin die Verbindung der Formel Ib 3-(3-Cyanobenzyliden)-2,4-pentandion ist.

22. Verwendung einer Verbindung, wie sie in einem der Ansprüche 6 und 9 bis 19 definiert worden ist, zur Herstellung eines Medikaments zur Behandlung oder Prophylaxe von Geschwüren oder Läsionen im gastrointestinalen Trakt.

23. Verwendung nach Anspruch 22, worin die Verbindung 3-(3-Cyanobenzyliden)-2,4-pentandion ist.

## Revendications (Revendications pour l'(les) Etat(s) contractant(s) suivant(s): AT, BE, CH, DE, FR, GB, IT, LI, LU, NL, SE)

1. Composé répondant à la formule Ib : 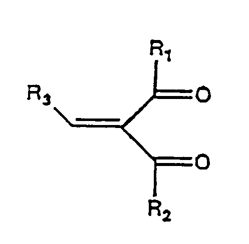 où R₁ et R₂ sont, indépendamment l'un de l'autre, un groupe alkyle en C₁₋₆, qui est non substitué ou substitué par un groupe hydroxy, alcoxy en C₁₋₆, halogéno, nitro ou amino ; un groupe alcoxy en C₁₋₆ ; un groupe phényle ou naphtyle qui est non substitué ou substitué par un groupe alkyle en C₁₋₆, hydroxy, alcoxy en C₁₋₆, amino ou carboxy ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
R₃ est un groupe alkyle en C₁₋₆ qui est substitué par un groupe hydroxy, alcoxy en C₁₋₆, halogéno, nitro ou amino ; un groupe phényle ou naphtyle qui est substitué par un groupe trifluorométhyle ou cyano ; ou un des groupes hétéroaryles suivants : quinolyle, thiényle ou furyle, qui sont substitués par un groupe alkyle en C₁₋₆, hydroxy, alcoxy en C₁₋₆, halogéno, nitro ou amino ;
ou un sel, un éther ou un ester de celui-ci, avec la condition que, quand R₃ est un groupe furyle substitué, il n'est pas substitué par un groupe alkyle, halogéno ou nitro, et si R₃ est un groupe phényle qui est substitué par un groupe cyano, alors le groupe cyano n'est pas en position para.

2. Composé selon la revendication 1, dans lequel R₁ et R₂ sont chacun un groupe alkyle en C₁₋₄.

3. 3-(4-trifluorométhylbenzylidène)-2,4-pentanedione.

4. 3-(3-cyanobenzylidène)-2,4-pentanedione.

5. Procédé de préparation d un composé Ib selon la revendication 1, comprenant la mise en réaction d'un composé de formule II :
R₁ - CO - CH₂ - CO - R₂ II
où R₁ et R₂ sont tels que définis dans la revendication 1, avec un composé de formule III :
R₃Z III
où R₃ est tel que défini dans la revendication 1, Z est CHO ou -CH₂-Q, où Q est un halogène ou un autre groupe activé, en présence d'un catalyseur acide ou basique, pour produire le composé Ia : 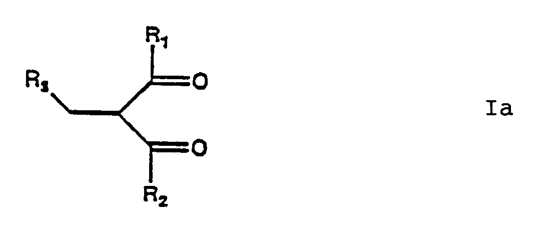 où R₁, R₂ et R₃ sont tels que définis dans la revendication 1, après quoi on halogène le composé de formule Ia pour donner un composé de formule IV : 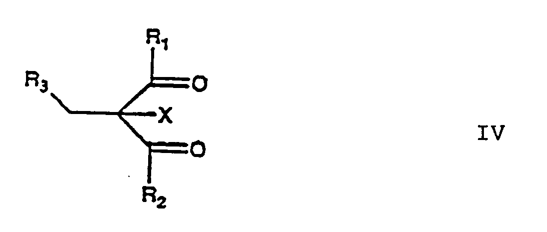 où R₁, R₂ et R₃ sont tels que définis dans la revendication 1, et X est un halogène,
et on soumet le composé IV à une déshydrohalogénation pour produire un composé de formule Ib.

6. Procédé selon la revendication 5, dans lequel le catalyseur basique est une base minérale ou une amine organique.

7. Procédé selon la revendication 5, dans lequel le catalyseur acide est un acide minéral ou un acide sulfonique.

8. Procédé selon l'une des revendications 5, 6 et 7, dans lequel l'agent d'halogénation est le chlore, le brome ou le chlorure de sulfuryle.

9. Composé de formule : 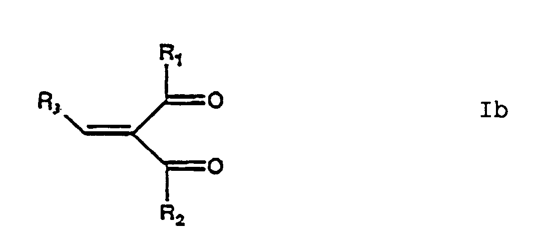 où R₁ et R₂ sont, indépendamment l'un de l'autre, un groupe alkyle en C₁₋₆, qui est non substitué ou substitué par un groupe hydroxy, alcoxy en C₁₋₆, halogéno, nitro ou amino ; un groupe alcoxy en C₁₋₆ ; un groupe phényle ou naphtyle qui est non substitué ou substitué par un groupe alkyle en C₁₋₆, hydroxy, alcoxy en C₁₋₆, amino ou carboxy ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
R₃ est un groupe alkyle en C₁₋₆ qui est non substitué ou substitué par un groupe hydroxy, alcoxy en C₁₋₆, halogéno, nitro ou amino ; un groupe phényle ou naphtyle qui est non substitué ou substitué par un groupe alkyle en C₁₋₆, alcoxy en C₁₋₆, trifluorométhyle, cyano, amino ou carboxy, ou un groupe dicéto attaché au groupe aryle, directement ou par l'intermédiaire d'une chaîne hydrocarbonée saturée contenant de 1 à 3 atomes de carbone ou d'une chaîne hydrocarbonée non saturée contenant 2 ou 3 atomes de carbone ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
ou un sel, un éther ou un ester de celui-ci, avec la condition que, quand R₁ et R₂ sont chacun un groupe méthyle ou l'hydrogène, alors R₃ ne peut pas être 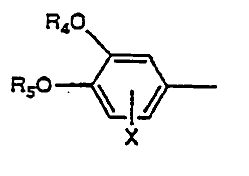 où R₄ et R₅ sont, indépendamment l'un de l'autre, un atome d'hydrogène ou un groupe alkyle, un groupe acyle éventuellement substitué ou un groupe aryle éventuellement substitué, un groupe (alkyle inférieur)sulfonyle ou alkylcarbamoyle, ou, lorsqu'ils sont pris ensemble, R₄ et R₅ forment un groupe alkylidène inférieur ou un groupe cycloalkylidène, et X est un groupe électronégatif, pour une utilisation comme un produit pharmaceutique.

10. Composé selon la revendication 9, dans lequel R₁ et R₂ sont chacun un groupe alkyle en C₁₋₄, et R₃ est un groupe phényle qui est non substitué ou substitué par un groupe alcoxy en C₁₋₄, carboxy, trifluorométhyle ou cyano, ou R₃ est un groupe thiényle ou furyle qui est non substitué ou substitué par un groupe alcoxy en C₁₋₄, trifluorométhyle ou cyano.

11. Composé selon les revendications 9 et 10 pour une utilisation dans le traitement ou la prophylaxie des ulcères ou des lésions de l'appareil gastro-intestinal.

12. 3-(4-méthoxybenzylidène)-2,4-pentanedione pour une utilisation dans le traitement ou la prophylaxie des ulcères ou des lésions de l'appareil gastro-intestinal.

13. 3-(benzylidène)-2,4-pentanedione pour une utilisation dans le traitement ou la prophylaxie des ulcères ou des lésions de l'appareil gastro-intestinal.

14. 3-éthylidène-2,4-pentanedione pour une utilisation dans le traitement ou la prophylaxie des ulcères ou des lésions de l'appareil gastro-intestinal.

15. Malonate de diméthylbenzylidène pour une utilisation dans le traitement ou la prophylaxie des ulcères ou des lésions de l'appareil gastro-intestinal.

16. 1,4-bis(2-acétyl-3-oxo-1-butényl)benzène pour une utilisation dans le traitement ou la prophylaxie des ulcères ou des lésions de l'appareil gastro-intestinal.

17. 3-acétyl-4-(2-thiényl)-3-butène-2-one pour une utilisation dans le traitement ou la prophylaxie des ulcères ou des lésions de l'appareil gastro-intestinal.

18. 3-acétyl-4-(2-furyl)-3-butène-2-one pour une utilisation dans le traitement ou la prophylaxie des ulcères ou des lésions de l'appareil gastro-intestinal.

19. 3-(4-cyanobenzylidène)-2,4-pentanedione pour une utilisation dans le traitement ou la prophylaxie des ulcères ou des lésions de l'appareil gastro-intestinal.

20. 3-(4-carboxybenzylidène)-2,4-pentanedione pour une utilisation dans le traitement ou la prophylaxie des ulcères ou des lésions de l'appareil gastro-intestinal.

21. 3-(2-trifluorométhylbenzylidène)-2,4-pentanedione pour une utilisation dans le traitement ou la prophylaxie des ulcères ou des lésions de l'appareil gastro-intestinal.

22. 3-(3-cyanobenzylidène)-2,4-pentanedione pour une utilisation dans le traitement ou la prophylaxie des ulcères ou des lésions de l'appareil gastro-intestinal.

23. Utilisation d'un composé tel que défini dans l'une quelconque des revendications 9 à 22 pour la fabrication d'un médicament pour le traitement ou la prophylaxie des ulcères ou des lésions de l'appareil gastro-intestinal.

24. Utilisation de la 3-benzylidène-2,4-pentanedione pour la fabrication d'un médicament pour le traitement ou la prophylaxie des ulcères ou des lésions de l'appareil gastro-intestinal.

25. Composition pharmaceutique comprenant un composé selon l une quelconque des revendications 1 à 4 ou tel que défini dans l'une quelconque des revendications 9 à 22, et un véhicule ou un diluant pharmaceutiquement acceptable.

26. Composition pharmaceutique comprenant de la 3-(3-cyanobenzylidène)-2,4-pentanedione et un véhicule ou un diluant pharmaceutiquement acceptable.

## Revendications (Revendications pour l'(les) Etat(s) contractant(s) suivant(s): ES, GR)

1. Procédé de préparation d un composé répondant à la formule Ib : 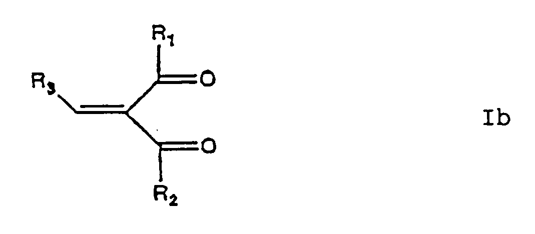 où R₁ et R₂ sont, indépendamment l'un de l'autre, un groupe alkyle en C₁₋₆, qui est non substitué ou substitué par un groupe hydroxy, alcoxy en C₁₋₆, halogéno, nitro ou amino ; un groupe alcoxy en C₁₋₆ ; un groupe phényle ou naphtyle qui est non substitué ou substitué par un groupe alkyle en C₁₋₆, hydroxy, alcoxy en C₁₋₆, amino ou carboxy ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
R₃ est un groupe alkyle en C₁₋₆ qui est substitué par un groupe hydroxy, alcoxy en C₁₋₆, halogéno, nitro ou amino ; un groupe phényle ou naphtyle qui est substitué par un groupe trifluorométhyle ou cyano ; ou un des groupes hétéroaryles suivants : quinolyle, thiényle ou furyle, qui sont substitués par un groupe alkyle en C₁₋₆, hydroxy, alcoxy en C₁₋₆, halogéno, nitro ou amino ;
ou un sel, un éther ou un ester de celui-ci, avec la condition que, quand R₃ est un groupe furyle substitué, il n'est pas substitué par un groupe alkyle, halogéno ou nitro, et si R₃ est un groupe phényle qui est substitué par un groupe cyano, alors le groupe cyano n'est pas en position para,
comprenant la mise en réaction d'un composé de formule II :
R₁ - CO - CH₂ - CO - R₂ II
où R₁ et R₂ sont tels que définis ci-dessus, avec un composé de formule III :
R₃Z III
où R₃ est tel que défini ci-dessus, Z est CHO ou -CH₂-Q, où Q est un halogène ou un autre groupe activé, en présence d'un catalyseur acide ou basique, pour produire le composé Ia : 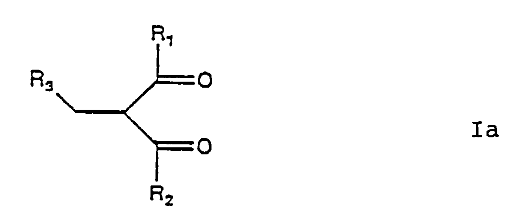 où R₁, R₂ et R₃ sont tels que définis ci-dessus, après quoi on halogène le composé de formule Ia pour donner un composé de formule IV : 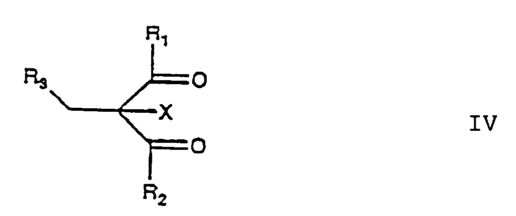 où R₁, R₂ et R₃ sont tels que définis ci-dessus, et X est un halogène, et on soumet le composé IV à une déshydrohalogénation pour produire un composé de formule Ib.

2. Procédé selon la revendication 1, dans lequel le catalyseur basique est une base minérale ou une amine organique.

3. Procédé selon la revendication 1, dans lequel le catalyseur acide est un acide minéral ou un acide sulfonique.

4. Procédé selon l'une des revendications 1, 2 et 3, dans lequel l'agent d'halogénation est le chlore, le brome ou le chlorure de sulfuryle.

5. Procédé selon l'une quelconque des revendications précédentes pour la préparation d'un composé où R₁ et R₂ sont chacun un groupe alkyle en C₁₋₄.

6. Procédé selon l'une quelconque des revendications 1 à 4 pour la préparation de 3-(2-trifluorométhylbenzylidène)-2,4-pentanedione.

7. Procédé selon l'une quelconque des revendications 1 à 4 pour la préparation de 3-(4-trifluorométhylbenzylidène)-2,4-pentanedione.

8. Procédé selon l'une quelconque des revendications 1 à 4 pour la préparation de 3-(3-cyanobenzylidène)-2,4-pentanedione.

9. Procédé de préparation d'une composition pharmaceutique, comprenant le mélange d'un composé de formule : 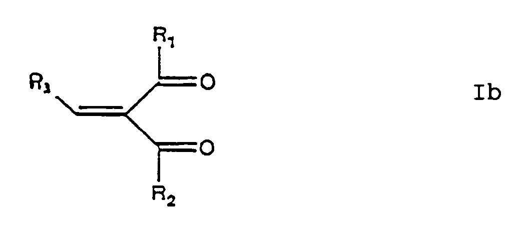 où R₁ et R₂ sont, indépendamment l'un de l'autre, un groupe alkyle en C₁₋₆, qui est non substitué ou substitué par un groupe hydroxy, alcoxy en C₁₋₆, halogéno, nitro ou amino ; un groupe alcoxy en C₁₋₆ ; un groupe phényle ou naphtyle qui est non substitué ou substitué par un groupe alkyle en C₁₋₆, hydroxy, alcoxy en C₁₋₆, amino ou carboxy ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
R₃ est un groupe alkyle en C₁₋₆ qui est non substitué ou substitué par un groupe hydroxy, alcoxy en C₁₋₆, halogéno, nitro ou amino ; un groupe phényle ou naphtyle qui est non substitué ou substitué par un groupe alkyle en C₁₋₆, alcoxy en C₁₋₆, trifluorométhyle, cyano, amino ou carboxy, ou un groupe dicéto attaché au groupe aryle, directement ou par l'intermédiaire d'une chaîne hydrocarbonée saturée contenant de 1 à 3 atomes de carbone ou d'une chaîne hydrocarbonée non saturée contenant 2 ou 3 atomes de carbone ; ou un des groupes hétéroaryles suivants : pyridyle, quinolyle, thiényle ou furyle, qui sont non substitués ou substitués par un groupe alkyle en C₁₋₆, hydroxy, alcoxy en C₁₋₆, halogéno, nitro ou amino ;
ou un sel, un éther ou un ester de celui-ci, avec la condition que, quand R₁ et R₂ sont chacun un groupe méthyle ou l'hydrogène, alors R₃ ne peut pas être 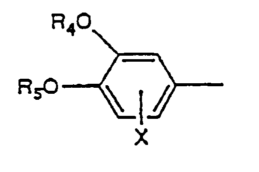 où R₄ et R₅ sont, indépendamment l'un de l'autre, un atome d'hydrogène ou un groupe alkyle, un groupe acyle éventuellement substitué ou un groupe aryle éventuellement substitué, un groupe (alkyle inférieur)sulfonyle ou alkylcarbamoyle, ou, lorsqu'ils sont pris ensemble, R₄ et R₅ forment un groupe alkylidène inférieur ou un groupe cycloalkylidène, et X est un groupe électronégatif, avec un véhicule ou un diluant pharmaceutiquement acceptable.

10. Procédé selon la revendication 9, dans lequel le composé de formule Ib est un composé où R₁ et R₂ sont chacun un groupe alkyle en C₁₋₄, et R₃ est un groupe phényle qui est non substitué ou substitué par un groupe alcoxy en C₁₋₄, carboxy, trifluorométhyle ou cyano, ou R₃ est un groupe thiényle ou furyle qui est non substitué ou substitué par un groupe alcoxy en C₁₋₄, trifluorométhyle ou cyano.

11. Procédé selon la revendication 9, dans lequel le composé de formule Ib est la 3-(4-méthoxybenzylidène)-2,4-pentanedione.

12. Procédé selon la revendication 9, dans lequel le composé de formule Ib est la 3-(benzylidène)-2,4-pentanedione.

13. Procédé selon la revendication 9, dans lequel le composé de formule Ib est la 3-éthylidène-2,4-pentanedione.

14. Procédé selon la revendication 9, dans lequel le composé de formule Ib est le malonate de diméthylbenzylidène.

15. Procédé selon la revendication 9, dans lequel le composé de formule Ib est le 1,4-bis(2-acétyl-3-oxo-1-butényl)benzène.

16. Procédé selon la revendication 9, dans lequel le composé de formule Ib est la 3-acétyl-4-(2-thiényl)-3-butène-2-one.

17. Procédé selon la revendication 9, dans lequel le composé de formule Ib est la 3-acétyl-4-(2-furyl)-3-butène-2-one.

18. Procédé selon la revendication 9, dans lequel le composé de formule Ib est la 3-(4-cyanobenzylidène)-2,4-pentanedione.

19. Procédé selon la revendication 9, dans lequel le composé de formule Ib est la 3-(4-carboxybenzylidène)-2,4-pentanedione.

20. Procédé selon la revendication 9 ou 10, dans lequel le composé de formule Ib est un Composé tel que défini dans l'une quelconque des revendications 1 et 5 à 8.

21. Procédé selon la revendication 9, dans lequel le composé de formule Ib est la 3-(3-cyanobenzylidène)-2,4-pentanedione.

22. Utilisation d'un composé tel que défini dans l'une quelconque des revendications 6 et 9 à 19 pour la fabrication d'un médicament pour le traitement ou la prophylaxie des ulcères ou des lésions de l'appareil gastro-intestinal.

23. Utilisation selon la revendication 22, où le composé est la 3-(3-cyanobenzylidène)-2,4-pentanedione.
